# EUROPEAN PATENT APPLICATION

(11) **EP 3 795 153 A1**
(43) Date of publication of application: **24.03.2021**
(21) Application number: 19803519.8
(22) Date of filing: 13.05.2019
(51) Int. Cl.: A61K 31/4166, A61K 9/48, A61P 35/00

(54) **HC-1119 FORMULATION, PREPARATION METHOD AND USE THEREOF**

(30) Priority: 14.05.2018 CN 201810458165
(71) Applicant: Hinova Pharmaceuticals Inc., Chengdu, Sichuan 610041 (CN)
(72) Inventor: WEI, Xing, Chengdu, Sichuan 610041 (CN); QI, Ming, Chengdu, Sichuan 610041 (CN); DU, Wu, Chengdu, Sichuan 610041 (CN); LI, Xinghai, Chengdu, Sichuan 610041 (CN); CHEN, Yuanwei, Chengdu, Sichuan 610041 (CN)
(74) Representative: Altmann Stößel Dick Patentanwälte PartG mbB
(86) International application number: PCT/CN2019/086694
(87) International publication number: WO 2019/218979

(57) **Abstract**

Provided is a formulation of androgen receptor inhibitor HC-1119 prepared from the following raw materials at the weight ratio: 1 to 100 parts of the androgen receptor inhibitor HC-1119, 100 to 1000 parts of a solvent, and 0.11 to 11 parts of an antioxidant. Also provided is a HC-1119 soft capsule and a HC-1119 formulation. The HC-1119 raw material is dissolved in Labrasol so as to significantly improve the solubility of HC-1119, and thus greatly improve the bioavailability, reduce the difference in blood drug concentration and exposure among individuals, and increase the safety of medication. Further, the HC-1119 soft capsule has good stability.

## Description

### Technical field

The present invention relates to a formulation of androgen receptor inhibitor HC-1119, preparation method and use thereof.

### Background technology

HC-1119 is an inhibitor of androgen receptor (AR), and it can competitively inhibit the binding of androgen and AR and block the transmission of AR signal pathway. The indication of HC-1119 is the androgen signal pathway dependent diseases, including but not limited to prostate cancer and breast cancer. Its chemical name is 4-{3-[4-cyano-3-(trifluoromethyl)phenyl]-5,5-dimethyl-4-oxo-2-thio-1-imidazolidinyl}-2 -fluoro-N- trideuteromethylbenzamide, with a structure of formula I:

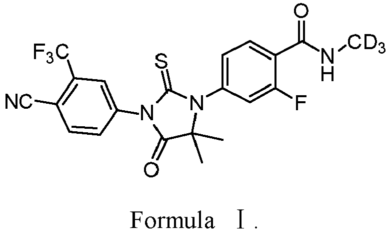

Application number: 201280052853.9, with an invention name: imidazolidinedione compounds and uses thereof, disclosed compound HC-1119.

The results of investigating on physical and chemical properties of HC-1119 show that HC-1119 is difficult to dissolve in water, and the results of *in vitro* experiments indicate that HC-1119 has the characteristics of high permeability (10 µM HC-1119 having an apparent permeability coefficient of 11.4 to 13.6× 10⁻⁶ cm/s), when the permeability of HC-1119 is evaluated by Caco-2 cell model. Therefore, HC-1119 is an insoluble drug with high permeability, and belongs to the second category of BSC classification, and thus it is extremely important to improve the bioavailability thereof.

### Content of the invention

In order to solve the above problems, the present invention provides a formulation of HC-1119, as well as a soft capsule containing HC-1119.

HC-1119 formulation according to the present invention is prepared from the following raw materials at the following weight ratio:
1 to 100 parts of androgen receptor inhibitor HC-1119, 100 to 1000 parts of solvent, and 0.11 to 11 parts of antioxidant.

Wherein, the solvent is a combination of one or more of Labrasol, soybean oil, glycerol, polyethylene glycol, polyethylene glycol glyceride, and caprylic/capric glyceride; preferably, said polyethylene glycol glyceride is selected from the group consisting of polyethylene glycol monoglyceride, polyethylene glycol triglyceride and a mixture of two thereof; preferably said caprylic/capric glyceride is caprylic/capric triglyceride; more preferably, the solvent is Labrasol.

Wherein, the antioxidant is a combination of one or more of butyl hydroxyanisole, butylhydroxytoluene and aromatic amine;
preferably, the antioxidant is the composition of butylhydroxyanisole and butylhydroxytoluene; wherein butylhydroxyanisole is 0.1 to 10 parts, and butylhydroxytoluene is 0.01 to 1 part.

Further, the formulation of HC-1119 mentioned above is preferably prepared from the following raw materials at the following weight ratio:
5 to 55 parts of androgen receptor inhibitor HC-1119, 460 to 980 parts of Labrasol, 0.25 to 1.5 parts of butyl hydroxyanisole, 0.025 to 0.15 parts of butyl hydroxytoluene.

Further, the formulation of HC-1119 mentioned above is more preferably prepared from the following raw materials at the following weight ratio:
25 to 55 parts of androgen receptor inhibitor HC-1119, 920 to 980 parts of Labrasol, 0.5 to 1.5 parts of butyl hydroxyanisole, 0.05 to 0.15 parts of butyl hydroxytoluene;
or, 15 to 25 parts of androgen receptor inhibitor HC-1119, 460 to 500 parts of Labrasol, 0.25 to 0.75 parts of butyl hydroxyanisole, 0.025 to 0.075 parts of butyl hydroxytoluene;
or, 5 to 15 parts of androgen receptor inhibitor HC-1119, 460 to 500 parts of Labrasol, 0.25 to 0.75 parts of butyl hydroxyanisole, 0.025 to 0.075 parts of butyl hydroxytoluene.

Further, the formulation of HC-1119 mentioned above is more preferably prepared from the following raw materials at the following weight ratio:
40 parts of androgen receptor inhibitor HC-1119, 958.9 parts of Labrasol, 1 part of butyl hydroxyanisole, 0.1 part of butyl hydroxytoluene;
or, 20 parts of androgen receptor inhibitor HC-1119, 479.5 parts of Labrasol, 0.5 part of butyl hydroxyanisole, 0.05 part of butyl hydroxytoluene;
or, 10 parts of androgen receptor inhibitor HC-1119, 479.5 parts of Labrasol, 0.5 part of butyl hydroxyanisole, 0.05 part of butyl hydroxytoluene;
or, 12 parts of androgen receptor inhibitor HC-1119, 271.5 parts of Labrasol, 0.03 part of butyl hydroxyanisole, 0.03 part of butyl hydroxytoluene.

Further, the formulation is prepared from the following raw materials in the following amount:
1 to 100 mg, preferably 80 mg, of androgen receptor inhibitor HC-1119;
or, 25 to 55 mg, preferably 40 mg, of androgen receptor inhibitor HC-1119;
or, 15 to 25 mg, preferably 20 mg, of androgen receptor inhibitor HC-1119;
or, 5 to 15mg, preferably 10 mg, of androgen receptor inhibitor HC-1119.

Wherein, the formulation is a tablet, a liquid formulation, and a soft capsule; preferably, the formulation is a soft capsule.

The present invention provides a soft capsule of androgen receptor inhibitor HC-1119, that is composed of a formulation mentioned above and a capsule shell;
the capsule shell is composed of the following excipients as the following weight ratio: 100 parts of gelatin, 20 to 60 parts of glycerol, 20 to 60 parts of sorbitol solution or 10 to 50 parts of sorbitol, 0.5 to 2 parts of titanium dioxide, and 50 to 100 parts of purified water.

The present invention further provides the preparation method of the formulation mentioned above, that includes the following steps:
(1) HC-1119 raw materials were micronized to have a particle size of 1 to 150 µm for use;
(2) under the protection of nitrogen, HC-1119 raw materials, Labrasol, butyl hydroxyanisole and butyl hydroxytoluene are mixed as the above weight ratio, stirred at 40 to 60 °Cuntil HC-1119 raw material is completely dissolved, and then vacuumized and replaced with nitrogen.

Wherein, the dissolution temperature in step (2) is 45 °C ± 3 °C.

The present invention further provide the preparation method of the soft capsule mentioned above, that include the following steps:
1) preparation of capsule shell:
   a) heating a rubber melting tank to 70 °C, and introducing water, glycerin and a sorbitol solution therein at the abovementioned weight ratios, and stirring for 20 min;
   b) taking out a part of the solution and adding titanium dioxide at the weight ratio mentioned above, then dispersing with a high-speed shear machine, and adding the uniformly dispersed solution to the rubber melting tank;
   c) adding gelatin at the abovementioned weight ratio, and dispersing to the viscous state;
   d) closing tightly the rubber tank, and vacuum degassing , then stirring for 1 h under a vacuum degree of -0.06 to -0.1 Mpa at a temperature of 50 to 60 °C, followed by staying overnight in non-vacuum state under insulation;
2) preparation of soft capsule:
   A) under the protection of nitrogen, controlling the thickness of rubber d from 0.8 mm to 1.10 mm, and the filing content of the capsule is ±5% of the theoretical content, then pressing the capsule;
   B) after pressing the capsule, blowing it to the drum by cold air and shaping. Adding an oil absorption cotton to wipe the pills, and drying the capsules at a temperature of 25.0 to 30.0 °C and a humidity of ≦25.0% until the moisture content of the content is less than 5.0%.

The present invention further provides the use of the formulation mentioned above in the preparation of a drug for the treatment of androgen signal pathway dependent diseases; preferably, the androgen signal pathway dependent diseases are prostate cancer and breast cancer; preferably, the prostate cancer is castration-resistant prostate cancer.

The present invention further provides the use of the soft capsule mentioned above in the preparation of a drug for the treatment of androgen signal pathway dependent diseases; preferably, the androgen signal pathway dependent diseases are prostate cancer and breast cancer; preferably, the prostate cancer is castration-resistant prostate cancer.

The present invention further provides an oral formulation of androgen receptor inhibitor HC-1119, that is an oral formulation prepared from an effective dose of HC-1119 as the active component and pharmaceutically acceptable excipients, and each formulation unit contains 1-100 mg of androgen receptor inhibitor HC-1119.

The formulation unit denotes the dosage form unit of conventional pharmaceutical formulation such as a tablet, a capsule, and a bag of granules.

Preferably, each formulation unit contains 80 mg of androgen receptor inhibitor HC-1119.

Preferably, each formulation unit contains 25 to 55 mg of androgen receptor inhibitor HC-1119, preferably 40 mg;
or, each formulation unit contains 15 to 25 mg of androgen receptor inhibitor HC-1119, preferably 20 mg;
or, each formulation unit contains 5 to 15 mg of androgen receptor inhibitor HC-1119, preferably 10 mg.

The present invention provides an oral formulation mentioned above for use in the preparation of a drug for the treatment of androgen signal pathway dependent diseases; preferably, the androgen signal pathway dependent diseases are prostate cancer and breast cancer; preferably, the prostate cancer is castration-resistant prostate cancer.

The present invention further provides the formulation mentioned above, the soft capsule mentioned above, or the oral formulation mentioned above for use in the treatment of androgen signaling pathway dependent diseases;
preferably, the androgen signal pathway dependent diseases are prostate cancer and breast cancer; preferably, the prostate cancer is castration-resistant prostate cancer.

The present invention further provides an androgen receptor inhibitor HC-1119 for use as a medicament, characterized in that 1 to 100 mg of said androgen receptor inhibitor HC-1119 are administrated to a patient, once a day, or twice a day, or once every two days, or once every three days, or once in four days, or once in five days, or once in six days, or once a week.

Preferably, 80 mg of said androgen receptor inhibitor HC-1119 are administrated to the patient, once a day.

Preferably, 25 to 55 mg, preferably 40 mg, of said androgen receptor inhibitor HC-1119 are administrated to the patients;
or, 15 to 25 mg, preferably 20 mg, of said androgen receptor inhibitor HC-1119 are administrated to the patient;
or, 5 to 15 mg, preferably 10 mg, of said androgen receptor inhibitor HC-1119 are administrated to the patient.

Preferably, the administration of said androgen receptor inhibitor HC-1119 is realized by giving the formulation mentioned above and the soft capsule mentioned above.

The present invention provides a method for treatment of androgen signaling pathway dependent diseases, in which patients are administrated 1 to 100 mg of androgen receptor inhibitor HC-1119, once a day.

Preferably, 80 mg of said androgen receptor inhibitor HC-1119 are administrated to the patient, once a day.

Preferably, 25 to 55 mg, preferably 40 mg, of said androgen receptor inhibitor HC-1119are administrated to the patient;
or, 15 to 25 mg, preferably 20 mg, of said androgen receptor inhibitor HC-1119 are administrated to the patient;
or, 5 to 15 mg, preferably 10 mg, of said androgen receptor inhibitor HC-1119 are administrated to the patient.

Preferably, the administration of androgen receptor inhibitor HC-1119 is realized by giving the formulation of HC-1119 mentioned above and the soft capsule mentioned above.

Wherein, the androgen signal pathway dependent diseases are prostate cancer and breast cancer; preferably, the prostate cancer is castration-resistant prostate cancer.

In the present invention, the sorbitol solution is purchased from SPI Pharma New Castle, and the model is Sorbitol Special™.

HC-1119 formulation of the present invention significantly improves the solubility of HC-1119 by dissolving the raw material of HC-1119 in Labrasol, greatly increases the bioavailability, reduces the difference in the blood trough concentration and the exposure between individuals, and enhances the safety of the drug. HC-1119 soft capsule of the present invention has good stability.

In clinical pharmacokinetic experiments, the formulation of the soft capsule shows that the exposure dose of the original drug enzalutamide can be reached with a half-dose, reduces the difference in the blood trough concentration (C_{trough}) and the exposure dose (AUC) between individuals, and improves the safety.

Obviously, based on above content of the present invention, according to the common technical knowledge and the conventional means in the field, without department from above basic technical spirits, other various modifications, alternations or changes can further be made.

By following specific examples of said embodiments, above content of the present invention is further illustrated. But it should not be construed that the scope of above subject of the present invention is limited to following examples. The techniques realized based on above content of the present invention are all within the scope of the present invention.

### Examples

The starting materials and equipment used in specific example of the present invention are all known products and can be obtained by purchasing commercially available products.

Wherein, the sorbitol solution is purchased from SPI Pharma New Castle, and the model is Sorbitol Special™.

Abbreviations in the present invention: CCMG (Labrasol), BHA (butyl hydroxyanisole), BHT (butyl hydroxytoluene).

### Example 1 Preparation of HC-1119 soft capsule of the present invention

### 1. Formula of raw and auxiliary materials

Formula of raw and auxiliary materials of the present invention was shown in Table 1.

**Table 1 Formula of raw and auxiliary materials of the present invention**

| HC-1119 soft capsules (specification: 40 mg/capsule) | | | |
|---|---|---|---|
| Formulation of the content | Names of raw and auxiliary materials | Percentage content of the raw materials in the formula (%) | Amount in the formula (g) |
| | HC-1119 | 4.0 | 400 |
| | CCMG | 95.89 | 9589 |
| | BHA | 0.1 | 10 |
| | BHT | 0.01 | 1 |

| Preparing 10000 capsules | | | |
|---|---|---|---|
| Capsule shell composition | Names of auxiliary materials | Percentage content of the raw materials in the formula (%) (Based on the amount of dry shell) | Amount in the formula (Kg) |
| | Gelatin | 62.05 | 10 |
| | Glycerol | 18.62 | 3 |
| | Sorbitol | 18.62 | 3 |
| | Titanium dioxide | 0.71 | 0.115 |
| | Purified water | - | 10 |
| Preparing an appropriate amount of gel (the amount of gel is determined by the volume of the gel tank) | | | |

### 2. Preparation method

### 1) Preparation of the capsule shell

① Sorbitol, glycerin, gelatin, and titanium dioxide were weighed according to the formula;
② The gel tank was turned on, and the temperature was kept at 65 to 70 °C;
③ About 70% of the formulated amount of purified water was taken out, to which was added the formulated amount of sorbitol, and stirred until it was completely dissolved;
④ Above sorbitol solution was transferred to the gel tank, and the stirring was turned on, then the solution was heated to 70 °C;
⑤ After removal of stirring, the formulated amount of glycerin was transferred to the gelatinization tank, and additional 30% of the formulated amount of purified water was gradually added to dissolve the remaining glycerin and transfer it to the gelatinization tank, meanwhile the stirring was continued until the dissolution was complete, then the tank was heated to 70 °C for 10 min;
⑥ After the stirring was stopped, a part of above solution was taken out, to which was added the formulated amount of titanium dioxide, and dispersed with a high-shear machine, then the dispersed solution was added to the gel tank;
⑦ The formulated amount of gelatin was slowly added to the gelatinization tank, the stirring was turned on and continued to obtain a uniform viscous gel;
⑧ The gelatinization tank was closely sealed, the vacuum degassing was turned on to keep the vacuum degree of -0.06 to -0.1 MPa, and stirred for 1 h, then the stirring and the vacuum was turned off, and the temperature of tank was kept at 60 °C overnight for use.

### 2) Content formulation

① Pretreatment: BHT and HC-1119 were micronized and passed through a sieve of 100 mesh;
② HC-1119, CCMG, HBA, and BHT were weighed according to the formulated amount;
③ The dosing tank was turned on, the temperature was kept at 45 °C ± 3 °C, and the formulated amount of BHT, BHA, and HC-1119 were successively placed in the dosing tank, then the formulated amount of CCMG was slowly added; the dosing tank was sealed, and nitrogen was purged for protection, then the stirring was turned on; when the solution became clear and transparent, the stirring was removed, and the tank was heated, followed by cooling to room temperature for use.

### 3) Formulation of soft capsule

① Pressing pill: the contents were transferred to the feed hopper, and nitrogen was continued to purge for protection, then pill was pressed. During the filling process, capsules with unqualified appearance should be selected at any time, and meanwhile the rubber thickness should be controlled from 0.8 mm to 1.10 mm, and the filling volume of the capsule content should be ± 5% of the theoretical filling volume.
② After the capsule was pressed, it was blown by cold air to the drum for drying. Oil-absorbent cotton was added to rub pill;
③ Sampling and determination: when the moisture of the content and the rubber skin reached about 8.0%, a drying process was stopped;
④ Drying capsule: the capsules were transferred to the drying room (temperature 20 to 25 °C, relative humidity 28 to 35%) for secondary drying, and the sample was collected and tested every 3 h until the moisture content of the capsule skin and the contents was less than 5.0%. Drying was stopped.

### Example 2 Preparation of HC-1119 soft capsule of the present invention

### 1. Formula of raw and auxiliary materials

Formula of raw and auxiliary materials of the present invention was shown in Table 2.

**Table 2 Formula of raw and auxiliary materials of the present invention**

| HC-1119 soft capsules (specification: 40 mg/capsule) | | | |
|---|---|---|---|
| Formulation of the content | Names of raw and auxiliary materials | Percentage content of materials in the formula (%) | Amount in the formula (g) |
| | HC-1119 | 4.0 | 400 |
| | CCMG | 95.89 | 9589 |
| | BHA | 0.1 | 10 |
| | BHT | 0.01 | 1 |

| Preparing 10000 capsules | | | |
|---|---|---|---|
| Capsule shell composition | Names of auxiliary materials | Percentage content of materials in the formula (%) (Based on the amount of dry shell) | Amount in the formula (Kg) |
| | Gelatin | 58.82 | 10 |
| | Glycerol | 17.68 | 3.005 |
| | Sorbitol | 22.82 | 3.88 |
| | Titanium dioxide | 0.68 | 0.115 |
| | Purified water | - | 9 |
| Preparing an appropriate amount of gel (the amount of gel is determined by the volume of the gel tank) | | | |

### 2. Preparative method

### 1) Formulation of capsule shell

① Sorbitol, glycerin, gelatin, and titanium dioxide were weighed according to the formula;
② The gel tank was turned on, and the temperature was kept at 70 °C; the formulated amount of purified water, sorbitol solution, and glycerin was added to the tank and stirred for 20 min to make the mixture uniform;
③ A part of above solution was taken out, to which was added the formulated amount of titanium dioxide, and dispersed with a high-shear machine, then the dispersed solution was added to the gel tank;
④ The formulated amount of gelatin was slowly added to the gelatinization tank, the stirring was turned on and continued to obtain a uniform viscous gel;
⑤ The gelatinization tank was closely sealed, the vacuum degassing was turned on to keep the vacuum degree of -0.06 to -0.1 MPa, and stirred for 1 h, then the stirring and the vacuum was turned off, and the temperature of tank was kept at 60 °C overnight for use.

### 2) Content formulation

① Pretreatment: HC-1119 was subjected to micronized treatment, D90 < 20 µm;
② HC-1119, CCMG, HBA, and BHT were weighed according to the formulated amount;
③ The following operations were finished under protection of nitrogen, the dosing tank was turned on, and added the formulated amount of CCMG (vacuum, nitrogen replacement), then heated to 45 °C±3 °C, and the formulated amounts of BHA and BHT were added under stirring;
④ HC-1119 was put into the dosing tank, and the stirring was turned on until the solution became clear and transparent, stirring and heating were stopped; and the tank was cooled to room temperature, and the content was filtered with 200 mesh sieve for use.

### 3) Formulation of soft capsule

① Adjustment of capsule skin thickness: gel was extrued, and the capsule skin thickness (1.00 to 1.10 mm) and the process parameters were adjusted;
② Cleaning the pipeline: CCMG was used for adjusting, and the process parameters and the loading amount were preliminarily adjusted at the same time;
③ Trying to press pill: After the process parameters are stable, the content was added to the feed hopper to clean the pipeline, and adjust the filling volume;
④ Pressing pill: The contents were transferred to the feed hopper, and nitrogen was continued to purge for protection, then pill was pressed. During the filling process, capsules with unqualified appearance should be selected at any time, and meanwhile the rubber thickness should be controlled from 0.8 mm to 1.10 mm, and the filling volume of the capsule content should be ±5% of the theoretical filling volume.
⑤ After the capsule was pressed, it was blown by cold air to the drum for drying. Oil-absorbent cotton was added to rub pill; after cleaning, the capsules were further dried. Drying conditions: the temperature being 25.0 to 30.0 °C, and the humidity being ≦ 25.0%. The sample was collected and tested until the moisture content of the capsule skin and the contents was less than 5.0%, and drying was stopped.

### Example 3 Preparation of HC-1119 soft capsule of the present invention

| HC-1119 soft capsules (specification: 40 mg/capsule) | | | |
|---|---|---|---|
| Formulation of the content | Names of raw and auxiliary materials | Percentage content of materials in the formula (%) | Amount in the formula (g) |
| | HC-1119 | 4.23 | 12g |
| | CCMG | 95.75 | 271.5g |
| | BHA | 0.01 | 30mg |
| | BHT | 0.01 | 30mg |

| Preparing 300 capsules | | | |
|---|---|---|---|
| Capsule shell composition | Names of auxiliary materials | Percentage content of ingredient in the formula (%) (Based on the amount of dry shell) | Amount in the formula (Kg) |
| | Gelatin | 58.69 | 2990.0 |
| | Glycerol | 17.88 | 910.8 |
| | Sorbitol | 22.75 | 1159.2 |
| | Titanium dioxide | 0.68 | 34.5 |
| | Purified water | - | 2990.0 |
| Preparing an appropriate amount of gel (the amount of gel is determined by the volume of the gel tank) | | | |

### 2. Preparative method

### 1) Formulation of capsule shell

① Sorbitol solution, glycerin, gelatin, and titanium dioxide were weighed according to the formula;
② The gel tank was turned on, and the temperature was kept at 70 °C; the formulated amount of purified water, sorbitol solution, and glycerin was added to the tank and stirred for 20 min to make the mixture uniform;
③ A part of above solution was taken out, to which was added the formulated amount of titanium dioxide, and dispersed with a high-shear machine, then the dispersed solution was added to the gel tank;
④ The formulated amount of gelatin was slowly added to the gelatinization tank, and the stirring was turned on and continued to obtain a uniform viscous gel;
⑤ The gelatinization tank was closely sealed, the vacuum degassing was turned on to keep the vacuum degree of -0.06 to -0.1 MPa, and stirred for 1 h, then the stirring and the vacuum was turned off, and the temperature of tank was kept at 60 °C overnight for use.

### 2) Content formulation

① Pretreatment: HC-1119 was subjected to micronized treatment, D90 < 20 µm;
② HC-1119, CCMG, HBA, and BHT were weighed according to the formulated amount;
③ The following operations were finished under protection of nitrogen, the dosing tank was turned on, and added the formulated amount of CCMG (vacuum, nitrogen replacement), then heated to 45 °C±3 °C, and the formulated amounts of BHA and BHT were added under stirring;
④ HC-1119 was put into the dosing tank, and the stirring was turned on until the solution became clear and transparent, stirring and heating were stopped; and the tank was cooled to room temperature, and the content was filtered with 200 mesh sieve for use.

### 3) Formulation of soft capsule

① Adjustment of capsule skin thickness: gel was extrued, and the capsule skin thickness (1.00 to 1.10 mm) and the process parameters were adjusted;
② Cleaning the pipeline: CCMG was used for adjusting, and the process parameters and the loading amount were preliminarily adjusted at the same time;
③ Trying to press pill: After the process parameters are stable, the content was added to the feed hopper to clean the pipeline, and adjust the filling volume;
④ Pressing pill: The contents were transferred to the feed hopper, and nitrogen was continued to purge for protection, then pill was pressed. During the filling process, capsules with unqualified appearance should be selected at any time, meanwhile, the difference in the loading amount and the skin thickness was monitored, and both should be controlled within ± 5%. If abnormalities are found, they should be adjusted in time.
⑤ After the capsule was pressed, it was blown by cold air to the drum for drying. Oil-absorbent cotton was added to rub pill; after cleaning, the capsules were further dried. Drying conditions: the temperature being 25.0 to 30.0 °C, and the humidity being ≦ 25.0%. The sample was collected and tested until the moisture content of the capsule skin and the contents was less than 5.0%, and drying was stopped.

### Example 4 Preparation of HC-1119 soft capsule of the present invention

| HC-1119 soft capsules (specification: 20 mg/capsule) | | | |
|---|---|---|---|
| Formulation of the content | Names of raw and auxiliary materials | Percentage content of ingredient in the formula (%) | Amount in the formula (g) |
| | HC-1119 | 4.0 | 200 |
| | CCMG | 95.89 | 4794.5 |
| | BHA | 0.1 | 5 |
| | BHT | 0.01 | 0.5 |

| Preparing 10000 capsules | | | |
|---|---|---|---|
| Capsule shell composition | Names of auxiliary materials | Percentage content of ingredient in the formula (%) (Based on the amount of dry shell) | Amount in the formula (Kg) |
| | Gelatin | 58.82 | 100 |
| | Glycerol | 17.68 | 30.05 |
| | Sorbitol | 22.82 | 38.8 |
| | Titanium dioxide | 0.68 | 1.15 |
| | Purified water | - | 100 |
| Preparing an appropriate amount of gel (the amount of gel is determined by the volume of the gel tank) | | | |

### 2. Preparation method

### 1) Formulation of capsule shell

① Sorbitol solution, glycerin, gelatin, and titanium dioxide were weighed according to the formula;
② The gel tank was turned on, and the temperature was kept at 70 °C; the formulated amount of purified water, sorbitol solution, and glycerin was added to the tank and stirred for 20 min to make the mixture uniform;
③ A part of above solution was taken out, to which was added the formulated amount of titanium dioxide, and dispersed with a high-shear machine, then the dispersed solution was added to the gel tank;
④ The formulated amount of gelatin was slowly added to the gelatinization tank, and the stirring was turned on and continued to obtain a uniform viscous gel;
⑤ The gelatinization tank was closely sealed, the vacuum degassing was turned on to keep the vacuum degree of -0.06 to -0.1 MPa, and stirred for 1 h, then the stirring and the vacuum was turned off, and the temperature of tank was kept at 60 °C overnight.

### 2) Content formulation

① Pretreatment: HC-1119 was subjected to micronized treatment, D90 < 20 µm;
②HC-1119, CCMG, HBA, and BHT were weighed according to the formulated amount;
③ The following operations were finished under protection of nitrogen, the dosing tank was turned on, and added the formulated amount of CCMG (vacuum, nitrogen replacement), then heated to 45 °C±3 °C, and the formulated amounts of BHA and BHT were added under stirring;
④ HC-1119 was put into the dosing tank, and the stirring was turned on until the solution became clear and transparent, stirring and heating were stopped; and the tank was cooled to room temperature, then the content was filtered with 200 mesh sieve for use.

### 3) Formulation of soft capsule

① Adjustment of capsule skin thickness: gel was extrued, and the capsule skin thickness (1.00 to 1.10 mm) and the process parameters were adjusted;
② Cleaning the pipeline: CCMG was used for adjusting, and the process parameters and the loading amount were preliminarily adjusted at the same time;
③ Trying to press pill: After the process parameters are stable, the content was added to the feed hopper to clean the pipeline, and adjust the filling volume;
④ Pressing pill: The contents were transferred to the feed hopper, and nitrogen was continued to purge for protection, then pill was pressed. During the filling process, capsules with unqualified appearance should be selected at any time, meanwhile, the difference in the loading amount and the skin thickness was monitored, and both should be controlled within ± 5%. If abnormalities are found, they should be adjusted in time.
⑤ After the capsule was pressed, it was blown by cold air to the drum for drying. Oil-absorbent cotton was added to rub pill; after cleaning, the capsules were further dried. Drying conditions: the temperature being 25.0 to 30.0 °C, and the humidity being ≦ 25.0%. The sample was collected and tested until the moisture content of the capsule skin and the contents was less than 5.0%, and drying was stopped.

The beneficial effects of the present invention were illustrated by the following experimental examples:

### Experimental example 1 Stability test of HC-1119 soft capsule of the present invention

### 1. Experimental method

Stability test protocol: stability test was performed on the soft capsules produced in Example 2, and the stability test was carried out for accelerated 6 months and long-term 12 months, respectively. Three conditions were investigated, namely accelerated test (40 °C ± 2 °C, relative humidity (RH) 75% ± 5%); intermediate condition test (30 °C ± 2 °C, RH 65% ± 5%), and long-term test ( 25 °C ± 2 °C, RH 60% ± 5%). The package was aluminum-plastic blister with a middle-sealed bag outside. The properties, acid value, peroxide value, related substances, dissolution rate, antioxidant dose, moisture, microbial limit and content of soft capsules were investigated at each time point.

**Descriptions:** The detection method was visually observed. The standard for the product was a white oval capsule, and the content was light yellow oily liquid, without precipitation.

**Related substances:** the contents of the product were detected according to the HPLC method (Chinese Pharmacopoeia 2015 Edition General Rule 0512).

**Antioxidant dosage:** the contents of the product were detected according to the HPLC method (Chinese Pharmacopoeia 2015 Edition General Rule 0512).

**Dissolution:** The product was tested according to the dissolution test method (General Rule 0931 Second Method), in which hydrochloric acid solution containing 0.2% sodium lauryl sulfate (900 ml) was used as the dissolution medium, the rotary speed was set at 50 RPM, and the procedures were performed according to the method, then at 30 min, a suitable amount of solution was taken out for determination.

**Peroxide value:** the contents of this product was determined according to the method for determination of peroxide value in the fat and fatty oil determination method (General Rule 0713).

**Acid value:** the content of this product was determined according to the acid value determination method in the Fat and Fatty Oil Determination Method (General Rule 0713).

Moisture: the content of this product was determined according to the moisture determination method (Chinese Pharmacopoeia 2015 Edition General Rules 0832 First Method 1).

**Microbial limit:** the product was determined according to the microbial limit inspection of non-sterile products (General Rules 1105, 1106 and 1107).

**Determination of content:** the content was determined according to HPLC method (Chinese Pharmacopoeia 2015 Edition General Rule 0512).

**Chromatographic conditions and system suitability test:** octadecylsilane-bonded silica gel was used as loading materials; phosphoric acid aqueous solution (pH 3.0)-methanol-acetonitrile was used as mobile phase, and liquid chromatography was used to determine.

### 2. Experimental results:

(1) The first stability test conditions (accelerated test): the packaging was commercially available, and the investigation conditions were 40 °C ± 2 °C and RH 75% ± 5%. The accelerated test results were shown in Table 1.

**Table 1. The accelerated test results of HC-1119 soft capsules according to the present invention.**

| Investigation items | | Day 0 | 3 months | 6 months |
|---|---|---|---|---|
| Descriptions | | Off-white oval capsules, and the content was light yellow oily liquid, without precipitation. | Off-white oval capsules, and the content was light yellow oily liquid, without precipitation. | Off-white oval capsules, and the content was light yellow oily liquid, without precipitation. |
| Related substances (%) | Degraded impurities | 0.042 | 0.070 | 0.079 |
| | Total impurities | 0.22 | 0.26 | 0.27 |
| Antioxidants (%) | BHA | 0.09 | - | 0.09 |
| | BHT | 0.009 | - | 0.010 |
| Moisture (%) | | 3.9 | 4.0 | 4.0 |
| Acid value | | 0.70 | - | 0.85 |
| Peroxide value | | 0.33 | - | 0.11 |
| Dissolution (%) | | 97.2 | 99.8 | 98.2 |
| Microbial Limit | | Meeting the requirements | - | Meeting the requirements |
| Content (%) | | 97.8 | 98.3 | 97.6 |

(2) The second stability test conditions (intermediate condition test): the packaging was commercially available, and the investigation conditions are 30 °C ± 2 °C and RH 65% ± 5%. The test results of the intermediate conditions were shown in Table 2.

**Table 2. The intermediate conditions test results of HC-1119 soft capsules according to the present invention.**

| Investigation items | | Day 0 | 1 month | 2 months | 3 months | 6 months | 9 months | 12 months |
|---|---|---|---|---|---|---|---|---|
| Descriptions | | Off-whit e oval capsules, and the content was light yellow oily liquid, | Off-whit e oval capsules, and the content was light yellow oily liquid, | Off-white oval capsules, and the content was light yellow oily liquid, | Off-white oval capsules, and the content was light yellow oily liquid, | Off-white oval capsules, and the content was light yellow oily liquid, | Off-whit e oval capsules, and the content was light yellow oily liquid, | Off-whit e oval capsules , and the content was light yellow oily |

| | | without precipitation. | without precipitation. | without precipitation. | without precipitation. | without precipitation. | without precipitation. | liquid, without precipitation. |
|---|---|---|---|---|---|---|---|---|
| Relate d substa nces (%) | Degrad ed impurities | 0.042 | 0.048 | 0.055 | 0.059 | 0.071 | 0.069 | 0.069 |
| | Total impurities | 0.22 | 0.23 | 0.24 | 0.24 | 0.26 | 0.25 | 0.25 |
| Antio xidant (%) | BHA | 0.09 | - | - | - | 0.09 | 0.060 | - |
| | BHT | 0.009 | - | - | - | 0.010 | 0.25 | - |
| Moisture (%) | | 3.9 | 4.1 | 4.0 | 4.2 | 4.1 | 4.0 | 4.4 |
| Acid value | | 0.70 | - | - | - | 0.73 | - | - |
| Peroxide value | | 0.33 | - | - | - | 0.08 | - | - |
| Dissolution (%) | | 97.2 | 99.7 | 97.4 | 96.9 | 94.6 | 97.4 | 96.3 |
| Microbial Limit | | Meeting the requirem ents | - | - | - | - | - | - |
| Content (%) | | 97.8 | 97.5 | 97.7 | 97.3 | 98.9 | 96.4 | 96.6 |

(3) The third stability test conditions (long-term test): the packaging was commercially available, and the investigation conditions are 25 °C ± 2 °C and RH 60% ± 5%. The results of the long-term test were shown in Table 3.

**Table 3. The long-term test results of HC-1119 soft capsules according to the present invention.**

| Investigation items | | Day 0 | 3 months | 6 months | 9 months | 12 months |
|---|---|---|---|---|---|---|
| Descriptions | | Off-white oval capsules, and the content was light yellow oily liquid, without precipitation | Off-white oval capsules, and the content was light yellow oily liquid, without precipitation | Off-white oval capsules, and the content was light yellow oily liquid, without precipitation | Off-white oval capsules, and the content was light yellow oily liquid, without precipitation | Off-white oval capsules, and the content was light yellow oily liquid, without precipitation |
| Related substan ces(%) | Degraded impurities | 0.042 | 0.049 | 0.060 | 0.065 | 0.064 |
| | Total impurities | 0.22 | 0.23 | 0.25 | 0.25 | 0.24 |
| Antioxi dant (%) | BHA | 0.09 | - | 0.09 | - | - |
| | BHT | 0.009 | - | 0.010 | - | - |
| Moisture (%) | | 3.9 | 4.1 | 4.1 | 4.4 | 4.3 |
| Acid value | | 0.70 | - | 0.69 | - | - |
| Peroxide value | | 0.33 | - | 0.09 | - | - |
| Dissolution (%) | | 97.2 | 98.0 | 98.5 | 97.5 | 99.4 |
| Microbial Limit | | Meeting the requirements | - | - | - | - |
| Content (%) | | 97.8 | 97.1 | 98.2 | 97.2 | 97.0 |

After 6 months accelerated, 12 months intermediate conditions and 12 months long-term investigation, under the conditions of current packaging, the tested quality indicators of HC-1119 soft capsules were within the limits set by the quality standards. There was no obvious difference in related substances, content, dissolution, and descriptions compared with month 0, and thus the product was stable.

### Experimental example 2. Clinical pharmacokinetic test of HC-1119 soft capsule of the present invention

### 1. Mode of administration

Use HC-1119 soft capsules prepared in Example 2.
(1) Dosage: 80 mg/day (2 soft capsules of 40 mg)
(2) Administration way: continuous administration, once a day, the first administration being orally taken with empty stomach, thereafter no restriction on diet.

### 2. Experimental method

In the pharmacokinetic study, 9 subjects participated in this study.
1) Biological sample collection after single-dose:
   The drug was taken by the subjects with empty stomach on the first day of the test, and 0∼0.5 h before taking the drug as well as 0.5 h, 0.75 h, 1 h, 2 h (±5 min), 4 h, 8 h, 12 h (±15 min), 24 h (± 30 min) before administration on the second day), blood samples were collected, and 3 mL venous blood was drawn at each time point, then placed in an anticoagulant tube (the type of anticoagulant was determined according to the established analytical method).
2) Biological sample collection after continuous dosing:
   0∼0.5 h before administration on day 7, 21, 35, 42, 56, 70, and 84 of continuous dosing, as well as 0.5 h, 0.75 h, 1 h, 2 h (± 5 min), 4 h, 8 h, 12 h (±15min), 24 h (±30 min, before administration on day 85) after administration on day 84, 3 mL venous blood was collected at each time point and placed in an anticoagulant tube (the type of anticoagulant was determined according to the established analytical method).
3) Sample treatment:
   The sample should be centrifuged as soon as possible (about 3000 rpm, 10 min) to separate the plasma. The separated plasma samples need to be stored in two parts in a plasma cryotube. The subject's entry number, the subject's initials, and the date and time of collection were marked on the label. Plasma samples should be stored as soon as possible in a refrigerator at -60°C∼-80°C. The first part of blood sample would be kept in the research center, and the second part of blood sample would be transported to a professional testing company for determination of plasma drug concentration.

### 3. Experimental results

The clinical pharmacokinetic data of 80 mg HC-1119 soft capsule according to the present invention are shown in Table 4.

**Table 4 The clinical pharmacokinetic parameters of HC-1119 soft capsule according to the present invention (2x40 mg soft capsules, QD 84 days)**

| **HC-1119 soft capsules of the present invention** | | | | | | | | | |
|---|---|---|---|---|---|---|---|---|---|
| **Parameters** | **Day 1 Cmax (ng/mL)** | **Day 1 Tmax (h)** | **Day 1 AUC0-24h (h^{∗}ng/mL)** | **Day 84 Cmax_ss (ng/mL)** | **Day 84 C24h (ng/mL)** | **Day 84 Tmax_ss (h)** | **Day 84 Ctrough (ng/mL)** | **Day 84_ AUClast (h^{∗}ng/mL)** | **Day 84 PTR** |
| Number of samples | 9 | 9 | 9 | 8 | 8 | 8 | 8 | 8 | 8 |
| Mean values | 2841.11 | 1.00 | 29107.70 | 22912.50 | 20250.00 | 1993.50 | 18900.00 | 479745.31 | 1.22 |
| SD (CV%) | 523.87 (18.44) | 0.60 (59.95) | 8787.27 (30.19) | 3026.28 (13.21) | 3053.34 (15.08) | 1.56 (0.08) | 2744.34 (14.52) | 67360.15 (14.04) | 0.07 (5.46) |

The clinical pharmacokinetic data of enzalutamide (160 mg) published by FDA are shown in Table 5.

**Table 5 The clinical pharmacokinetic data of enzalutamide (160 mg QD 49 days)**

| **Enzalutamide** | | | | | | | |
|---|---|---|---|---|---|---|---|
| **Parameters** | **AUCₜₐᵤ (h^{∗}µg/mL)** | **C**₀ₕ **(µg/mL)** | **Cmin (µg/mL)** | **Cmax (µg/mL)** | **tmax (h)** | **CL/F (L/h)** | **PTR** |
| Number of samples | 14 | 14 | 14 | 14 | 14 | 14 | 14 |
| Mean values | 321.5 | 13.32 | 12.00 | 16.59 | NA | 0.520 | 1.266 |
| SD (CV%) | 85.39 (26.6) | 3.341 (25.1) | 3.512 (29.3) | 3.812 (23.0) | NA | 0.0942 (18.1) | 0.1271 (10.0) |
| Min-Max | 240-593 | 9.86-23.4 | 6.92-22.4 | 11.8-28.0 | 0.52-3.02 | 0.27-0.67 | 1.09-1.51 |
| Median | 295.6 | 12.70 | 11.45 | 15.55 | 1.02 | 0.541 | 1.240 |

HC-1119 was deuterated enzalutamide, and the *in vitro* biological activities of both were the same. However, in clinical pharmacokinetic experiments, HC-1119 soft capsules showed that when the dose was halved, 80 mg of HC-1119 could reach the mother drug exposure and the blood concentration not less than those of 160 mg enzalutamide. For HC-1119 (80 mg), the steady-state Ctrough = 18.9 µg/mL on day 84, which was higher than the steady-state Cmin = 12 µg/mL of enzalutamide (160 mg). For HC-1119 (80 mg), the steady-state AUC on day 84 = 479.7 h^{∗}µg/mL, which was higher than the steady-state AUC of enzalutamide (160 mg) = 321.5 h^{∗}µg/mL. HC-1119 soft capsule could reduce the dosage, improve the safety of the drug and the compliance of the patient.

At the same time, HC-1119 soft capsule reduced the fluctuation of the drug's pharmacokinetics among experimental subjects. The data fluctuation degree was represented by the standard coefficient of variation (CV). For HC-1119 (80 mg), the steady-state Ctrough on day 84 corresponded to CV = 14.5%, which was lower than CV = 29.3% of enzalutamide (160 mg) corresponding to the steady-state Cmin. For HC-1119 (80 mg), the steady-state AUC on day 84 corresponded to CV = 14%, which was lower than CV = 26.6% of enzalutamide (160 mg) corresponding to the steady-state AUC. Lower CV meant that the pharmacokinetics of HC-1119 soft capsules had little fluctuation among different patients, which could reduce the risks of efficacy and safety caused by individual differences in patients. HC-1119 soft capsule thus further improved the safety of the drug.

In summary, HC-1119 formulation of the present invention, by dissolving the raw material of HC-1119 in a soft capsule prepared from Labrasol, significantly improved the solubility of HC-1119, greatly increased the bioavailability, reduced the difference in the blood trough concentration and the exposure between individuals, and enhanced the safety of the drug. HC-1119 soft capsule of the present invention had a good stability.

## Claims

1. A formulation of an androgen receptor inhibitor HC-1119, **characterized in that** it is produced from the following raw materials at the following weight ratio: 1 to 100 parts of the androgen receptor inhibitor HC-1119, 100 to 1000 parts of a solvent, and 0.11 to 11 parts of an antioxidant.

2. The formulation according to claim 1, **characterized in that** the solvent is a combination of one or more of Labrasol, soybean oil, glycerol, polyethylene glycol, polyethylene glycol glyceride, and caprylic/capric glyceride;
preferably, wherein said polyethylene glycol glyceride is selected from of the group consisting of polyethylene glycol monoglyceride, polyethylene glycol triglyceride and a mixture of two thereof; said caprylic/capric glyceride is selected from the caprylic/capric triglyceride;
more preferably, the solvent is Labrasol.

3. The formulation according to claim 1, **characterized in that** the antioxidant is a combination of one or more of butylhydroxyanisole, butylhydroxytoluene and aromatic amine;
preferably, the antioxidant is the combination of butylhydroxyanisole and butylhydroxytoluene; wherein butylhydroxyanisole is 0.1 to 10 parts, and butylhydroxytoluene is 0.01 to 1 part.

4. The formulation according to claim 1, **characterized in that** it is prepared from the following raw materials at the following weight ratio:
5to 55 parts of androgen receptor inhibitor HC-1119, 460 to 980 parts of Labrasol, 0.25 to 1.5 parts of butyl hydroxyanisole, 0.025 to 0.15 parts of butylhydroxytoluene.

5. The formulation according to claim 4, **characterized in that** it is produced from ingredients with the following weight ratio:
25 to 55 parts of androgen receptor inhibitor HC-1119, 920 to 980 parts of Labrasol, 0.5 to 1.5 parts of butylhydroxyanisole, and 0.05 to 0.15 parts of butyl hydroxytoluene;
or, 15 to 25 parts of androgen receptor inhibitor HC-1119, 460 to 500 parts of Labrasol , 0.25 to 0.75 parts of butylhydroxyanisole, and 0.025 to 0.075 parts of butyl hydroxytoluene;
or, 5 to 15 parts of androgen receptor inhibitor HC-1119, 460 to 500 parts of Labrasol, 0.25 to 0.75 parts of butyl hydroxyanisole, and 0.025 to 0.075 parts of butyl hydroxytoluene.

6. The formulation according to claim 4 or 5, **characterized in that** it is prepared from the following raw materials at the following weight ratio:
40 parts of the androgen receptor inhibitor HC-1119, 958.9 parts of Labrasol, 1 part of butyl hydroxyanisole, and 0.1 parts of butyl hydroxytoluene;
or, 20 parts of the androgen receptor inhibitor HC-1119, 479.5 parts of Labrasol, 0.5 parts of butyl hydroxyanisole, and 0.05 parts of butyl hydroxytoluene;
or, 10 parts of androgen receptor inhibitor HC-1119, 479.5 parts of Labrasol, 0.5 parts of butyl hydroxyanisole, and 0.05 parts of butyl hydroxytoluene;
or, 12 parts of androgen receptor inhibitor HC-1119, 271.5 parts of Labrasol, 0.03 parts of butyl hydroxyanisole, 0.03 parts of butyl hydroxytoluene.

7. The formulation according to claim 6, **characterized in that** it is prepared from the following raw materials in the following amount:
1 to 100 mg, preferably 80 mg, of the androgen receptor inhibitor HC-1119;
or, 25 to 55 mg, preferably 40 mg, of the androgen receptor inhibitor HC-1119;
or, 15 to 25 mg, preferably 20 mg, of the androgen receptor inhibitor HC-1119;
or, 5 to 15mg, preferably 10 mg, of the androgen receptor inhibitor HC-1119.

8. The formulation according to any one of claims 1 to 5 or 7, **characterized in that** the formulation is a tablet, a liquid formulation, and a soft capsule; preferably, the formulation is a soft capsule.

9. A soft capsule of androgen receptor inhibitor HC-1119, **characterized in that** it is composed of a formulation according to any one of claims 1 to 8 and a capsule shell; wherein the capsule shell is composed of the following excipients at the following weight ratio: 100 parts of gelatin, 20 to 60 parts of glycerol, 20 to 60 parts of sorbitol solution or 10 to 50 parts of sorbitol, 0.5 to 2 parts of titanium dioxide, and 50 to 100 parts of purified water.

10. The preparation method of the formulation according to any one of claims 1 to 8, **characterized in that** the method includes the following steps:
(1) crushing the HC-1119 raw material to a particle size of 1 to 150 µm ready for use;
(2) under nitrogen protection, mixing the HC-1119 raw material, Labrasol, butyl hydroxyanisole and butyl hydroxytoluene at the above weight ratio, stirring at 40 to 60°C until the HC-1119 raw material is completely dissolved, and then vacuum degassing and replacing with nitrogen.

11. The preparation method according claim 10, **characterized in that** the dissolution temperature in step (2) is 45 °C ± 3 °C.

12. The preparation method of the soft capsule according to claim 9, **characterized in that** it includes the following steps:
1) preparation of capsule shell
a) heating the rubber melting tank to 70 °C, and introducing water, glycerin and sorbitol solution therein at the above weight ratio, and stirring for 20 min;
b) taking out a part of the solution and adding titanium dioxide at the above weight ratio, then dispersing with a high-speed shear machine, and adding the dispersed solution to the rubber melting tank;
c) adding gelatin at the above weight ratio, and dispersing to the viscous state;
d) air tightly closing the rubber tank, and vacuum degassing, then stirring for 1 h under a vacuum degree of -0.06 to -0.1 Mpa at a temperature of 50 to 60 °C, followed by staying overnight in non-vacuum state under insulation;
2) Formulation of soft capsule
A) under nitrogen protection, controlling the thickness of rubber from 0.8 mm to 1.10 mm, and the content of capsule is ±5% of the theoretical content, then pressing the capsule;
B) after pressing the capsule is pressed, blowing it to the drum by cold air and shaping; adding an oil absorption cotton to wipe the pills, and drying the capsules at a temperature of 25.0 to 30.0 °C and a humidity of ≦ 25.0% until the moisture content of the contents is less than 5.0%.

13. The use of the formulation according to any one of claims 1 to 8 in the preparation of a drug for the treatment of androgen signal pathway dependent diseases; preferably, the androgen signal pathway dependent diseases are prostate cancer and breast cancer; preferably, the prostate cancer is castration-resistant prostate cancer.

14. The use of the soft capsule according to claim 9 in the preparation of a drug for the treatment of androgen signal pathway dependent diseases; preferably, the androgen signal pathway dependent diseases are prostate cancer and breast cancer; preferably, the prostate cancer is castration-resistant prostate cancer.

15. An oral formulation of androgen receptor inhibitor HC-1119, **characterized in that** it is an oral formulation prepared from an effective dose of HC-1119 as the active component and pharmaceutically acceptable excipients, and each formulation unit contains 1 to 100 mg of said androgen receptor inhibitor HC-1119.

16. The oral formulation according to claim 15, **characterized in that** each formulation unit contains 80 mg of said androgen receptor inhibitor HC-1119.

17. The oral formulation according to claim 15, **characterized in that** each formulation unit contains 25 to 55 mg, preferably 40 mg, of said androgen receptor inhibitor HC-1119;
or, each formulation unit contains 15 to 25 mg, preferably 20 mg, of said androgen receptor inhibitor HC-1119;
or, each formulation unit contains 5 to 15 mg, preferably 10 mg, of said androgen receptor inhibitor HC-1119.

18. The use of the oral formulation according to any one of claims 15 to 17 in the preparation of a drug for the treatment of androgen signal pathway dependent diseases; preferably, the androgen signal pathway dependent diseases are prostate cancer and breast cancer; preferably, the prostate cancer is castration-resistant prostate cancer.

19. A treatment of androgen signaling pathway dependent diseases, **characterized in that** it gives to the patient the formulation according to any one of claims 1-8, the soft capsule according to claim 9, or the oral formulation according to any one of claims 15-17; preferably, the androgen signal pathway dependent diseases are prostate cancer and breast cancer; preferably, the prostate cancer is castration-resistant prostate cancer.

20. An administration method of androgen receptor inhibitor HC-1119, **characterized in that** patients are administrated 1 to 100 mg of androgen receptor inhibitor HC-1119, once a day, or twice a day, or once every two days, or once every three days, or once in four days, or once in five days, or once in six days, or once a week.

21. The administration method according to claim 20, **characterized in that** patients are administrated 80 mg androgen receptor inhibitor HC-1119, once a day.

22. The administration method according to claim 20, **characterized in that** patients are administrated 25 to 55 mg, preferably 40 mg, of androgen receptor inhibitor HC-1119;
or, patients are administrated 15 to 25 mg, preferably 20 mg, of androgen receptor inhibitor HC-1119;
or, patients are administrated 5 to 15 mg, preferably 10 mg, of androgen receptor inhibitor HC-1119.

23. The administration method according to claims 20 to 22, **characterized in that** the administration of androgen receptor inhibitor HC-1119 is realized by giving the formulation according to any one of claims 1 to 8 and the soft capsule according to claim 9.

24. A method for treatment of androgen signaling pathway dependent diseases, **characterized in that** patients are administrated 1 to 100 mg of androgen receptor inhibitor HC-1119, once a day.

25. The method according to claim 24, **characterized in that** patients are administrated 80 mg androgen receptor inhibitor HC-1119, once a day.

26. The method according to claim 24, **characterized in that** patients are administrated 25 to 55 mg, preferably 40 mg, of androgen receptor inhibitor HC-1119;
or, patients are administrated 15 to 25 mg, preferably 20 mg, of androgen receptor inhibitor HC-1119;
or, patients are administrated 5 to 15 mg, preferably 10 mg, of androgen receptor inhibitor HC-1119.

27. The method according to claims 24 to 26, **characterized in that** the administration of androgen receptor inhibitor HC-1119 is realized by giving the formulation according to any one of claims 1 to 8 and the soft capsule according to claim 9.

28. The method according to claims 24 to 27, **characterized in that** the androgen signal pathway dependent diseases are prostate cancer and breast cancer; preferably, wherein the prostate cancer is castration-resistant prostate cancer.
